# EUROPEAN PATENT SPECIFICATION

(11) **EP 3 458 161 B1**
(45) Date of publication and mention of the grant of the patent: **08.04.2020**
(21) Application number: 17715505.8
(22) Date of filing: 10.04.2017
(51) Int. Cl.: A61Q 11/00, A61K 8/24, A61K 8/25

(54) **ORAL CARE COMPOSITION COMPRISING CALCIUM SILICATE**
MUNDPFLEGEZUSAMMENSETZUNG ENTHALTEND CALCIUMSILIKAT
COMPOSITION DE PROTECTION ORALE CONTENANT DE SILICATE DE CALCIUM

(30) Priority: 19.05.2016 WO PCT/CN2016/082601; 28.06.2016 EP 16176565
(43) Date of publication of application: 27.03.2019
(73) Proprietor: Unilever N.V., 3013 AL Rotterdam (NL); Unilever PLC, London, Greater London EC4Y 0DY (GB)
(72) Inventor: LI, Xiaoke, Shanghai 200335 (CN); LIU, Weining, Shanghai 200335 (CN); XING, Huaiyong, Shanghai 200335 (CN); ZHOU, Huanjun, Shanghai 200335 (CN)
(74) Representative: Tansley, Sally Elizabeth
(86) International application number: PCT/EP2017/058546
(87) International publication number: WO 2017/198392

(56) References cited:
- WO-A1-2015/036277
- WO-A2-2010/054494

## Description

### Technical Field of the Invention

The present invention relates to oral care compositions such as tooth pastes, powders, gums, mouthwashes and the like. In particular the present invention relates to an oral care composition comprising calcium silicate and a soluble calcium source that results in reducing sensitivity and/or remineralization of teeth. The invention also relates to the use of such compositions for treating tooth hypersensitivity and/or remineralizing of teeth of an individual.

### Background of the Invention

Tooth hypersensitivity is a temporary induced pain sensation that affects up to 20% of the adult population. Hypersensitive teeth may be sensitive to temperature, pressure or chemical action.

The dentin of the tooth generally contains channels, called tubules, which provide for an osmotic flow between the inner pulp region of the tooth and the outer root surfaces. Tooth hypersensitivity may be related to the general increase in exposed root surfaces of teeth as a result of periodontal disease, toothbrush abrasion, or cyclic loading fatigue of the thin enamel near the dentin-enamel junction. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

There are different approaches to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

There is a continuing need for treating tooth hypersensitivity with a more efficient approach. The present inventors have now found unexpectedly that an oral care composition comprising calcium silicate, a soluble calcium source and a phosphate source provides excellent tubule blocking efficacy to reduce tooth sensitivity. Additionally, it has been further found that such oral care composition also shows excellent tooth remineralization efficacy.

### Additional Information

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

The additional information above does not describe an oral care composition comprising from 3 to 80% by weight of calcium silicate, a soluble calcium source and a phosphate source, wherein the calcium silicate and the soluble calcium source is present in a weight ratio from 1:3 to 20:1, and especially such oral care composition can treat tooth hypersensitivity and/or enhance tooth remineralization efficacy. Further related oral care compositions are disclosed in: WO2010/054494, WO2015/036277.

### Tests and Definitions

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purpose of the present invention means a paste or gel dentifrice for use with a toothbrush. Especially preferred are tooth pastes suitable for cleaning teeth by brushing for about two minutes.

### Particle Size

"Particle size" for the purpose of the present invention means D50 particle size. The D50 particle size of a particulate material is the particle size diameter at which 50 wt% of the particles are larger in diameter and 50 wt% are smaller in diameter. For the purpose of the present invention, particle sizes and distribution are measured using Malvern Mastersizer 2000 and Malvern ZetaSizer Nano series.

### Deposition Aid

"Deposition aid" for the purpose of the present invention means a material which aids deposition of actives such as calcium silicate and/or other benefit agents from the continuous phase of the composition onto the tooth surfaces during use of the composition. Benefit agents as used herein means an active typically delivered to human teeth and/or the oral cavity including the gums to enhance or improve a characteristic of those dental tissues.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular the pH may be measured by manually mixing 5 g oral care composition with 20 mL water for 30 s, then immediately testing the pH with indicator or a pH meter.

### Solubility

"Soluble" and "insoluble" for the purpose of the present invention means the solubility of a source (e.g., like calcium salts) in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Sparingly soluble", therefore, is defined to mean a source that dissolves in water to give a solution with a concentration of greater than 0.001 moles per litre and less than 0.1 moles per litre.

### Water of Hydration

"Water of hydration" for the purpose of the present invention means water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

### Substantially Free

"Substantially free of' for the purpose of the present invention means less than 1.5%, and preferably less than 1.0%, and more preferably less than 0.75% and more preferably still less than 0.5%, and even more preferably less than 0.1% and most preferably from 0.0 to 0.01% by weight, based on total weight of the oral care composition, including all ranges subsumed therein.

### Dual-Phase

"Dual-Phase" for the purpose of the present invention means a composition having two independent phases which are physically separate.

### Anhydrous Composition

"Anhydrous composition" for the purpose of the present invention means the water content of the composition is less than 1.5%, preferably from 0.0 to 0.75% by total weight of the oral care composition.

### Viscosity

Viscosity of a tooth paste is the value taken at room temperature (25°C) with a Brookfield Viscometer, Spindle No.4 and at a speed of 5 rpm. Values are quoted in centipoises (cP=mPa.s) unless otherwise specified.

### Remineralization

"Remineralization" for the purpose of the present invention means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Summary of the Invention

In a first aspect, the present invention is directed to an oral care composition comprising:
a) from 3 to 80% by weight of calcium silicate;
b) a soluble calcium source;
c) a phosphate source; and
d) a physiologically acceptable carrier;
wherein the calcium silicate and the soluble calcium source is present in a weight ratio from 1:3 to 20:1.

In a second aspect, the present invention is directed to a packaged oral care product comprising the oral care composition of the first aspect of this invention.

In a third aspect, the present invention is directed to a method of reducing sensitivity and/or remineralization of teeth of an individual comprising the step of applying the oral care composition of any embodiment of the first aspect to at least one surface of the teeth of the individual.

All other aspects of the present invention will more readily become apparent upon considering the detailed description and examples which follow.

### Detailed Description

Now it has been found that an oral care composition comprising calcium silicate, a soluble calcium source and a phosphate source can efficiently seal exposed dentinal tubules, providing excellent tubule blocking efficacy to reduce tooth sensitivity. Additionally, it has also been found that such oral care composition also shows excellent tooth remineralization efficacy.

### CALCIUM SILICATE

In a preferred embodiment, the calcium silicate used is CaSiO₃ which has low water solubility and is made commercially available under the name Sorbosil CA40 by PQ Corporation. In another preferred embodiment, the calcium silicate is insoluble, present as the composite material calcium oxide-silica (CaO-SiO₂), which is described, for example, in intemational patent application published as WO 2008/01517(Unilever) which is hereby incorporated by reference in its entirety. For a calcium silicate composite material, the atom ratio of calcium to silicon (Ca:Si) may be from 1:30 to 3:1. The Ca:Si ratio is preferably 1:20 to 2:1, and more preferably, from 1:10 to 1:1, and most preferably, from about 1:7 to 1 :1.5. The calcium silicate may comprise mono-calcium silicate, bi-calcium silicate, or tri-calcium silicate. The calcium silicate may be in a crystalline or amorphous state or even in a mesoporous state.

In addition to calcium oxide, silica, the particles comprising the calcium silicate which is not hydrated may comprise other components, such as metal cations, anions (such as phosphate) and the like. However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In another preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%.

The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate may be amorphous or at least partly crystalline or mesoporous. The calcium silicate is preferably particulate as this allows for maximum surface area for contact with dental tissue. Thus preferably the composition comprises particles comprising the calcium silicate. Preferably from 10 to 100%, and especially, from 25 to 100%, and most especially, from 70% to 100% by weight of the particles comprising calcium silicate used in this invention have a particle size from 100 nm to less than 50 microns, preferably from 500 nm to 30 microns, more preferably from 1 micron to 20 microns, most preferably from 3 micron to 15 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the oral care composition of the present invention comprises from 3 to 80% by weight of the calcium silicate, more preferably from 3 to 50%, most preferably from 5 to 30%, based on the total weight of the oral care composition and including all ranges subsumed therein.

### SOLUBLE CALCIUM SOURCE

The soluble calcium source suitable for use in this invention is limited only to the extent that the same may be used in the mouth.

Illustrative yet non-limiting examples of the types of soluble calcium source that may be used in this invention include, for example, calcium chloride, calcium nitrate, calcium acetate, calcium lactate, calcium gluconate, calcium formate, calcium malate, calcium propionate, calcium butyrate, calcium bicarbonate, calcium glycerophosphate, calcium ascorbate, mixtures thereof or the like. In a preferred embodiment, the calcium source is calcium chloride, calcium nitrate or mixtures thereof.

Typically, the oral care composition of the present invention comprises from 0.1 to 20% by weight of the soluble calcium source, more preferably from 1 to 15%, most preferably from 2 to 10%, based on the total weight of the oral care composition and including all ranges subsumed therein.

The oral care composition preferably comprises the calcium silicate and the soluble calcium source in a weight ratio from 1:3 to 20:1, more preferably from 1:3 to 10:1, most preferably from 1:1.5 to 5:1.

### PHOSPHATE SOURCE

The phosphate source that may be used in this invention is limited only to the extent that the same may be used in a composition suitable for use in the mouth. Illustrative examples of the types of phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like. The phosphate source is preferably one which is water soluble.

When used, the phosphate source typically makes up from 0.5 to 40%, and more preferably, from 1 to 30%, and most preferably, from 2 to 20% by weight of the oral care composition, based on total weight of the oral care composition and including all ranges subsumed therein. In a preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein. In another preferred embodiment, the phosphate source used is or at least comprises monosodium dihydrogen phosphate.

### OPTIONAL COMPONENTS

The oral care composition of the present invention is found to be effective in blocking dentinal tubules to reduce tooth sensitivity and inducing new hydroxyapatite layer formation on enamel surface. Without wishing to be bound by theory the present inventors believe that the presence of soluble calcium source increases the calcium ion concentration in the oral composition, which enhances the reaction between the calcium salts and phosphate source in the mouth to form calcium phosphate. The calcium phosphate precipitation can help the deposition of calcium silicate particles on surfaces of dentine and/or enamel. The calcium silicate may further react with phosphate source in the saliva and/or Si-OH groups of calcium silicate may have an affinity for Ca ions in teeth, which further enhances the tubule blocking efficacy and/or tooth remineralization efficacy.

The oral care composition of the present invention may further comprise a deposition aid. The deposition aid suitable for use in this invention is limited only to the extent that the same may be used in the mouth. In a preferred embodiment, the deposition aid provides plenty of calcium ions in the mouth and is inexpensive and abundant. In an especially preferred embodiment, the deposition aid is calcium salts in addition to the soluble calcium source which is included in the composition.

Illustrative yet non-limiting examples of the types of deposition aid that may be used in this invention include, for example, calcium sulfate hemihydrates, calcium dihydrogen phosphate, calcium aluminate, calcium monohydrogen phosphate or mixtures thereof or the like. In a preferred embodiment, the deposition aid is calcium dihydrogen phosphate, calcium sulfate hemihydrate or mixtures thereof.

It has been discovered that the materials used as deposition aids in this invention is biocompatible, and undergoes rapid reaction with water and complete resorption onto the tooth surface, therefore it can be used to aid the deposition of oral care actives such as calcium silicate and/or other benefit agents onto the tooth surface.

Typically, the oral care composition of the present invention comprises from 0.1 to 20% by weight of the deposition aid, more preferably from 0.2 to 15%, more preferably still from 0.5 to 10%, most preferably from 1 to 5%, based on the total weight of the oral care composition and including all ranges subsumed therein.

The oral care composition preferably comprises the calcium silicate and the deposition aid in a weight ratio from 20:1 to 1:5, more preferably from 15:1 to 1:3, most preferably from 10:1 to 1:1.

The composition of the present invention is an oral care composition and typically comprises physiologically acceptable carrier. The carrier preferably comprises at least surfactant, thickener, humectant or a combination thereof.

Preferably the oral care composition comprises a surfactant. Preferably the composition comprises at least 0.01 % surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants. The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of reducing sensitivity and/or remineralizing of teeth of an individual comprising applying the composition to at least one surface of the teeth of an individual. The oral care composition of this invention may additionally or alternatively be for use as a medicament and/or used in the manufacture of a medicament for providing an oral care benefit as described herein, such as for reducing sensitivity of the teeth of an individual. Alternatively and preferably, the use is non-therapeutic.

Preferably, the oral care composition is substantially free of water to prevent the premature reaction between the calcium salts and the phosphate source.

In a preferred embodiment, the oral care composition is a monophase anhydrous composition.

In another preferred embodiment, the oral care composition is a dual-phase composition comprising a calcium phase and a phosphate phase, wherein the calcium silicate and the soluble calcium source are present in the calcium phase and the phosphate source is present in the phosphate phase. The two phases are physically separate from one another by being in independent phases. The delivery of the two independent phases to the teeth may be simultaneous or sequential. In a preferred embodiment, the phases are delivered simultaneously.

Typically, the dual-phase composition is delivered by a dual-tube having a first compartment for calcium phase and a second compartment for phosphate phase, which allows for coextrusion of the two phases.

In a preferred embodiment, such a dual-tube has one of the compartments surrounding the other. In such embodiments, one phase is present as a sheath, surrounding the other phase in the core. In an especially preferred embodiment, the core is the calcium phase and the sheath is the phosphate phase.

In another preferred embodiment, such a dual-tube has the two compartments side by side within the same tube. In such embodiments, the two phases are extruded from the tube as one, such extrusion being termed "contact extrusion". A pump head may be used in such a dual-tube for squeezing the two phases from the tube as one.

The dual-phase oral care composition may be a gel composition that comprises two independent gel phases, the first is the calcium phase and the second is the phosphate phase. The means of delivery may involve a cotton rod, or a tray, onto which the calcium phase and the phosphate phase are applied, prior to the tray being placed in contact with the teeth.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day. When the oral care composition is a dual-phase composition, the two phases of the composition are mixed during application. The mixed phases are typically left on the teeth for from 3 minutes to 10 hours, more preferably from 3 minutes to 8 hours. The application may be carried out daily.

The following examples are provided to facilitate an understanding of the present invention. The examples are not provided to limit the scope of the claims.

### Examples

### Example 1

This example demonstrates the improved blockage of dentinal tubules by using soluble calcium source in combination with calcium silicate. All ingredients are expressed by weight percent of the total formulation, and as level of active ingredient.

**TABLE 1**

| Ingredient | Samples | | | |
|---|---|---|---|---|
| | **1** | **2** | **3** | **4** |
| Calcium silicate^{a} | 15 | 10 | 10 | 10 |
| Hydroxyapatite | -- | 5 | -- | -- |
| Calcium carbonate | -- | -- | 5 | -- |
| Calcium chloride | -- | -- | -- | 5 |
| Trisodium phosphate | 3.8 | 3.8 | 3.8 | 3.8 |
| Monosodium dihydrogen phosphate | 3.2 | 3.2 | 3.2 | 3.2 |
| Glycerin | 78 | 78 | 78 | 78 |

| | | | | |
|---|---|---|---|---|
| a. Commercially available calcium silicate (CaSiO₃) from P.Q.Corporation (Sorbosil CA40) | | | | |

### Methods

To evaluate blockage efficacy of dentinal tubules, the test sample was mixed with water at a ratio of 1g to 2 mL water to make a slurry. Sample 1 comprising only commercial calcium silicate was used as control.

Human dentine disks were eroded by 37% phosphoric acid for 1 min, then they were treated with different slurries via brushing following the same protocol. Eight human dentine disks were separated into four groups (n=2). The dentine disks were brushed with the slurry under a tooth brushing machine equipped with toothbrushes. The load of the tooth brushing was 170 g +/-5 g and the automatic brushing operated at a speed of 150 rpm. After brushing for 1 min, the dentine disks were soaked in toothpaste slurry for 1 min. Then the dentine disks were rinsed with distilled water and placed in simulated oral fluid (SOF) under the condition of a shaking water bath at 37°C and 60.0 rpm. After soaking for about 3 to 4 hours, the dentine disks were brushed with the slurry by machine using the same procedure as in the first step. The brushing was repeated three times for one day, then the dentine disks were kept in SOF ovemight(>12 hours) in a shaking water bath at 37°C to mimic oral environment. The dentine samples were brushed for 7 times.

Simulated oral fluid was made by combining the ingredients in Table 2:

**TABLE 2**

| Ingredient | Amount/g |
|---|---|
| NaCl | 16.07 |
| NaHCO₃ | 0.7 |
| KCl | 0.448 |
| K₂HPO₄^{∗}3H₂O | 3.27 |
| MgCl₂^{∗}6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

### Results

After 7 brushings, SEM (Scanning Electron Microscopy) images of the dentine disks were taken. It clearly showed that the dentinal tubules of dentine disks treated with Sample 4 comprising soluble calcium source were extensively blocked. For all other samples, it can be seen that lots of dentinal tubules were still open.

### Example 2

This example demonstrates the concentration of soluble calcium source can affect the tubule blockage efficacy. All ingredients are expressed by weight percent of the total formulation, and as level of active ingredient.

**TABLE 3**

| Ingredient | Samples | | | | |
|---|---|---|---|---|---|
| | **5** | **6** | **7** | **8** | **9** |
| Calcium silicate^{a} | 15 | 10 | 5 | 3.33 | - |
| Calcium chloride | - | 5 | 10 | 11.67 | 15 |
| Trisodium phosphate | 3.8 | 3.8 | 3.8 | 3.8 | 3.8 |
| Monosodium dihydrogen phosphate | 3.2 | 3.2 | 3.2 | 3.2 | 3.2 |
| Glycerin | 78 | 78 | 78 | 78 | 78 |

**TABLE 4**

| Ingredient | Samples | | |
|---|---|---|---|
| | **6** | **10** | **11** |
| Calcium silicate^{a} | 10 | 30 | 50 |
| Calcium chloride | 5 | 5 | 5 |
| Trisodium phosphate | 3.8 | 3.8 | 3.8 |
| Monosodium dihydrogen phosphate | 3.2 | 3.2 | 3.2 |
| Glycerin | 78 | 58 | 38 |

### Methods

### Samples Preparation

The same protocol was used to treat human dentine disks as described in Example 1. The dentine samples were brushed for 3 and 7 times.

### Scoring Standard for Tubules Blockage

Regardless of the original shape of the dentine disks, a square (with a size of 4mm x 4mm) is selected and one image is captured under 50x magnification. Within this square, five spots (each with a size of 150 µm x 150 µm, one in the middle, and one in every corner) are selected and observed under 1000x magnification. The blockage of tubules are accessed following the standards described in Table 5. The measurement is carried out for the two dentine disks of each test group.

**TABLE 5**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

### Results

After 3 and 7 brushings, SEM images of the dentine disks were taken. The images were analysed and scored. The results are summarized in Table 6 (error represents standard deviation for duplicate measurements).

**TABLE 6**

| Tubules Blockage Score | **5** | **6** | **7** | **8** | **9** |
|---|---|---|---|---|---|
| 3 brushings^{a} | 1.1^{A} ± 0.32 | 4.8^{B} ± 0.42 | 3.6^{C} ± 0.70 | 1.4^{A} ± 0.52 | 1.2^{A} ± 0.42 |
| 7 brushings^{b} | 1.4^{A} ± 0.52 | 5.0^{B} ± 0.00 | 4.8^{B} ± 0.42 | 1.6^{A} ± 0.70 | 1.2^{A} ± 0.42 |

| | | | | | |
|---|---|---|---|---|---|
| a. Values with different letter are significantly different (P<0.01). b. Values with different letter are significantly different (P<0.01). | | | | | |

After 3 brushings, it showed that Sample 6 comprising a combination of 5% of calcium chloride and 10% of calcium silicate showed significantly better tubule blockage efficacy compared to Sample 7. Both of Samples 6 and 7 showed superior tubule blockage efficacy to Samples 5 and 9 comprising only calcium silicate or calcium chloride respectively. Sample 8 comprising a smaller weight ratio of calcium silicate to calcium chloride showed comparable tubule blockage efficacy to Samples 5 and 9.

After 3 brushings, Sample 6 also showed comparable tubule blockage efficacy compared to Samples 10 and 11 comprising a higher amount of calcium silicate. SEM images clearly showed that almost all the dentinal tubules were extensively blocked.

After 7 brushings, Samples 6 and 7 showed comparable tubule blockage efficacy. SEM images clearly showed that the dentinal tubules were extensively blocked, while for Samples 5 and 9, lots of tubules were still open in this short treatment period.

### Example 3

This example demonstrates the dentinal tubule blockage efficacy of different soluble calcium source. All ingredients are expressed by weight percent of the total formulation, and as level of active ingredient.

**TABLE 7**

| Ingredient | Samples | |
|---|---|---|
| | **12** | **13** |
| Calcium silicate^{a} | 10 | 10 |
| Calcium chloride | 5 | -- |
| Ca(NO₃)₂ • 4H₂O | -- | 10.6 |
| Trisodium phosphate | 3.8 | 3.8 |
| Monosodium dihydrogen phosphate | 3.2 | 3.2 |
| Glycerin | 78 | 72.4 |

### Methods

The same protocol was used to evaluate the blockage efficacy of dentinal tubules as described in Example 1. The dentine samples were brushed for 7 times.

### Results

After 7 brushings, SEM images of dentine disks showed that both Samples 12 and 13 had excellent tubule blockage efficacy. It can be seen that the dentinal tubules were extensively blocked.

### Example 4

This example demonstrates the improved blockage of dentinal tubules by using soluble calcium source in combination with calcium silicate in full formulations. All ingredients are expressed by weight percent of the total formulation, and as level of active ingredient.

**TABLE 8**

| Ingredient | Samples | | |
|---|---|---|---|
| | **14** | **15** | **16** |
| Glycerin | 64.84 | 64.84 | 66.84 |
| Sodium monofluorophosphate | 1.11 | 1.11 | 1.11 |
| Sodium saccharin | 0.25 | 0.25 | 0.25 |
| Calcium silicate^{a} | 15.00 | 10.00 | 10.00 |
| Calcium chloride | -- | 5.00 | 5.00 |
| Calcium dihydrogen phosphate | 2.00 | 2.00 | -- |
| Sodium lauryl sulfate | 2.00 | 2.00 | 2.00 |
| Monosodium dihydrogen phosphate | 3.20 | 3.20 | 3.20 |
| Trisodium phosphate | 3.80 | 3.80 | 3.80 |
| Silica abrasive^{b} | 6.00 | 6.00 | 6.00 |
| Silica abrasive^{c} | 0.50 | 0.50 | 0.50 |
| Xanthan gum | 0.10 | 0.10 | 0.10 |
| flavor | 1.20 | 1.20 | 1.20 |

| | | | |
|---|---|---|---|
| b. Commercially available silica under the trade name Sorbosil AC77 from PQ Corporation. c. Commercially available silica under the trade name Sorbosil AC43 from PQ Corporation. | | | |

### Methods

The same protocol was used to evaluate the blockage efficacy of dentinal tubules as described in Example 1. The dentine samples were brushed for 3 and 7 times.

### Results

After 3 brushings, SEM images of dentine disks were taken. They showed that Samples 15 and 16 comprising soluble calcium source like calcium chloride had much better tubule blockage efficacy compared to Sample 14. Sample 15 showed comparable tubule blockage efficacy to Sample 16, which further comprises calcium dihydrogen phosphate.

After 7 brushings, all three samples showed similar results, dentinal tubules were extensively blocked. But the corresponding cross-section SEM images after 7 brushings showed that for Samples 15 and 16, the materials penetrated deep into the dentinal tubules and the tubules were completely filled. While for sample 14, the materials were only deposited on top of the dentinal tubules in this short treatment period.

### Example 5

This example demonstrates the improved deposition on tooth surface by using soluble calcium source in combination with calcium silicate in full formulations. Samples used here were Samples 14-16 as listed in Example 4.

### Methods

To evaluate deposition on tooth surfaces, the test sample was mixed with water at a ratio of 1g to 2 mL water to make a toothpaste slurry.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 1 min, the enamel blocks were soaked in toothpaste slurry for 1 min. Then the enamel blocks were rinsed with distilled water and placed in SOF under the condition of a shaking water bath at 37°C and 60.0 rpm. After soaking for about 3 to 4 hours, the enamel blocks were brushed with the slurry by machine using the same procedure as in the first step. The brushing was repeated three times for one day, then the enamel blocks were kept in SOF ovemight(>12 hours) in a shaking water bath at 37°C to mimic oral environment. The enamel blocks were brushed for 14 times.

### Results

After 14 brushings, SEM images of the enamel block surfaces were taken. From the top view of the SEM images, it was apparent that Samples 15 and 16 gave better and denser deposition on tooth surface than Sample 14. The corresponding cross-section SEM images further showed that a new layer was formed on the surface of the enamel blocks after treatment with Samples 15 and 16, while the enamel blocks treated with Sample 14 did not have a layer formed on their surfaces in this short treatment period. Analysis using EDX (Energy Dispersive X-ray Spectroscopy) identified the elements of Si, Ca and P within the new layer, indicating that calcium silicate deposited on tooth surface and induced remineralization.

### Example 6

This example demonstrates the effect of the concentration of soluble calcium source in full formulations on dentinal tubule blockage efficacy. All ingredients are expressed by weight percent of the total formulation, and as level of active ingredient.

**TABLE 9**

| Ingredient | Samples | | | |
|---|---|---|---|---|
| | **17** | **18** | **19** | **20** |
| Glycerin | 70.84 | 68.84 | 66.84 | 64.84 |
| Sodium monofluorophosphate | 1.11 | 1.11 | 1.11 | 1.11 |
| Sodium saccharin | 0.25 | 0.25 | 0.25 | 0.25 |
| Calcium silicate^{a} | 10.00 | 10.00 | 10.00 | 10.00 |
| Calcium chloride | 1.00 | 3.00 | 5.00 | 7.00 |
| Sodium lauryl sulfate | 2.00 | 2.00 | 2.00 | 2.00 |
| Monosodium dihydrogen phosphate | 3.20 | 3.20 | 3.20 | 3.20 |
| Trisodium phosphate | 3.80 | 3.80 | 3.80 | 3.80 |
| Silica abrasive^{b} | 6.00 | 6.00 | 6.00 | 6.00 |
| Silica abrasive^{c} | 0.50 | 0.50 | 0.50 | 0.50 |
| Xanthan gum | 0.10 | 0.10 | 0.10 | 0.10 |
| flavor | 1.20 | 1.20 | 1.20 | 1.20 |

### Methods

The same protocol was used to evaluate the blockage efficacy of dentinal tubules as described in Example 1. The dentine samples were brushed for 3 times.

### Results

After 3 brushings, SEM images of dentine disks were taken. Sample 18 comprising 3% of calcium chloride showed much better tubule blockage efficacy compared to Sample 17 comprising just 1 % of calcium chloride. From the top view of the SEM images, the dentinal tubules were almost blocked. Samples 19 and 20 comprising higher concentration of calcium chloride showed even better tubule blockage efficacy.

### Example 7

This example demonstrates the effect of the concentration of soluble calcium source in full formulations on deposition on tooth surfaces. Samples used here were Samples 17-20 as listed in Example 6.

### Methods

The same protocol was used to evaluate deposition on tooth surfaces as described in Example 5. The enamel blocks were brushed for 3 times.

### Results

After 3 brushings, SEM images of the enamel block surfaces were taken. From the top view of the SEM images, it can be seen that all samples gave good deposition on tooth surfaces. But Samples 18-20 comprising higher concentration of calcium chloride showed better and denser deposition on tooth surfaces than Sample 17. The corresponding cross-section SEM images further showed that a new layer was formed on the surface of the enamel blocks after treatment with Samples 18-20, while the enamel blocks treated with Sample 17 did not have a layer formed on their surfaces in this short treatment period.

### Example 8

This example demonstrates the affinity of the newly formed layer on tooth surfaces. Samples used here were Sample 14 in Example 4 and Sample 18 in Example 6. All ingredients are expressed by weight percent of the total formulation, and as level of active ingredient.

**TABLE 10**

| Ingredient | Samples | |
|---|---|---|
| | **14** | **18** |
| Glycerin | 64.84 | 68.84 |
| Sodium monofluorophosphate | 1.11 | 1.11 |
| Sodium saccharin | 0.25 | 0.25 |
| Calcium silicate^{a} | 15.00 | 10.00 |
| Calcium chloride | -- | 3.00 |
| Calcium dihydrogen phosphate | 2.00 | -- |
| Sodium lauryl sulfate | 2.00 | 2.00 |
| Monosodium dihydrogen phosphate | 3.20 | 3.20 |
| Trisodium phosphate | 3.80 | 3.80 |
| Silica abrasive^{b} | 6.00 | 6.00 |
| Silica abrasive^{c} | 0.50 | 0.50 |
| Xanthan gum | 0.10 | 0.10 |
| flavor | 1.20 | 1.20 |

### Methods

To evaluate the affinity of the newly formed layer on tooth surfaces, the test sample was mixed with water at a ratio of 1 g to 2 mL water to make a toothpaste slurry.

The bovine enamel blocks were treated with different slurries via brushing following the same protocol. The enamel blocks were brushed with the slurry under a tooth brushing machine equipped with toothbrushes. The load of the tooth brushing was 170 g +/-5 g and the automatic brushing operated at a speed of 150 rpm. After brushing for 1 min, the enamel blocks were soaked in toothpaste slurry for 1 min. Then the enamel blocks were rinsed with distilled water and placed in SOF under the condition of a shaking water bath at 37°C and 60.0 rpm. After soaking for about 3 to 4 hours, the enamel blocks were brushed with water using the tooth brushing machine. The load of the tooth brushing was 170 g +/-5 g and the automatic brushing operated at a speed of 150 rpm. After brushing for 1 min, the enamel blocks were placed in SOF under the condition of a shaking water bath at 37°C and 60.0 rpm. After soaking for about 3 to 4 hours, the enamel blocks were brushed with water again using the tooth brushing machine for 1 min (170 g +/-5 g load, 150 rpm). Then the enamel blocks were brushed with fresh toothpaste slurry using the tooth brushing machine for 1 min (170 g +/-5 g load, 150 rpm) and soaked in toothpaste slurry for 1 min. Then the enamel blocks were rinsed with distilled water and kept in SOF overnight (>12 hours) in a shaking water bath at 37°C to mimic oral environment. The enamel blocks were then brushed with water using the tooth brushing machine for 1 min (170 g +/-5 g load, 150 rpm). These steps are considered as a whole treatment cycle for one day. The enamel blocks were treated for 7 days.

### Results

After 7 days of treatments, SEM images of the enamel block surfaces were taken. From the top view of the SEM images, it can be seen that Sample 18 showed better and denser deposition on tooth surfaces than Sample 14.

The corresponding cross-section SEM images further showed that a new layer was formed on the surfaces of the enamel blocks after treatment with Sample 18, while the enamel blocks treated with Sample 14 did not have a layer formed on their surfaces in this short treatment period. The results clearly showed that the newly formed layer was not brushed away by water brushing, indicating enhanced affinity of the newly formed layer on tooth surfaces. A section of the newly formed layer was collected by FIB (Focus Ions Beam) technique and then housed on TEM (Transmission Electron Microscopy) grid for SAED (Selected Area Electron Diffraction) and EDX characterization. Analysis using EDX identified the elements of Si, Ca and P within the new layer, indicating that calcium silicate deposited on tooth surfaces and induced remineralization. From the electron diffraction pattern obtained by SAED, it clearly showed that the newly formed layer on enamel blocks was hydroxyapatite (HAP).

## Claims

1. An oral care composition comprising:
a) from 3 to 80% by weight of calcium silicate;
b) a soluble calcium source;
c) a phosphate source; and
d) a physiologically acceptable carrier;
wherein the calcium silicate and the soluble calcium source is present in a weight ratio from 1:3 to 20:1.

2. The oral care composition according to claim 1, wherein the soluble calcium source is calcium chloride, calcium nitrate, calcium acetate, calcium lactate, calcium gluconate, calcium formate, calcium malate, calcium propionate, calcium butyrate, calcium bicarbonate calcium glycerophosphate, calcium ascorbate or mixtures thereof, preferably calcium chloride, calcium nitrate or mixtures thereof.

3. The oral care composition according to claim 1 or claim 2, wherein the phosphate source is water soluble.

4. The oral care composition according to any of the preceding claims, wherein the calcium silicate is calcium silicate hydrate which comprises water of hydration in an amount of from 5 to 50% by weight of the calcium silicate hydrate.

5. The oral care composition according to any of the preceding claims, wherein the calcium silicate is present in an amount from 3 to 50% by weight of the composition, preferably from 5 to 30%.

6. The oral care composition according to any of the preceding claims, wherein the calcium silicate and the soluble calcium source is present in a weight ratio from 1:3 to 10:1, preferably from 1:1.5 to 5:1.

7. The oral care composition according to any of the preceding claims, wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate or a mixture thereof; preferably trisodium phosphate, monosodium dihydrogen phosphate or a mixture thereof.

8. The oral care composition according to any of the preceding claims, wherein the phosphate source is present in an amount from 0.5 to 40% by weight of the composition, preferably from 1 to 30%.

9. The oral care composition according to any of the preceding claims, wherein the composition further comprises a deposition aid selected from calcium dihydrogen phosphate, calcium sulfate hemihydrates, calcium aluminate, calcium monohydrogen phosphate or a mixture thereof.

10. The oral care composition according to claim 9, wherein the deposition aid is calcium dihydrogen phosphate.

11. The oral care composition according to claim 9 or claim 10, wherein the calcium silicate and the deposition aid is present in a weight ratio from 10:1 to 1:1.

12. The oral care composition according to any of the preceding claims, wherein the oral care composition is a monophase anhydrous composition.

13. The oral care composition according to any of the preceding claims 1 to 11, wherein the composition is a dual-phase composition comprising a calcium phase and a phosphate phase, wherein the calcium silicate and the soluble calcium source is present in the calcium phase and the phosphate source is present in the phosphate phase.

14. The oral care composition according to claim 13, wherein the calcium phase and the phosphate phase are physically separate prior to the use of the composition.

15. A composition as claimed in any one of claims 1 to 14 for use for reducing sensitivity and/or for remineralizing of the teeth of an individual comprising the step of applying said composition to at least one surface of the teeth of the individual.

## Patentansprüche

1. Mundpflegezusammensetzung, umfassend:
a) von 3 bis 80 Gewichts-% Calciumsilikat;
b) eine lösliche Calciumquelle;
c) eine Phosphatquelle; und
d) einen physiologisch verträglichen Träger;
wobei das Calciumsilikat und die lösliche Calciumquelle in einem Gewichtsverhältnis von 1:3 bis 20:1 vorliegen.

2. Mundpflegezusammensetzung nach Anspruch 1, wobei die lösliche Calciumquelle Calciumchlorid, Calciumnitrat, Calciumacetat, Calciumlactat, Calciumgluconat, Calciumformiat, Calciummalat, Calciumpropionat, Calciumbutyrat, Calciumbicarbonat, Calciumglycerophosphat, Calciumascorbat oder Mischungen davon, vorzugsweise Calciumchlorid, Calciumnitrat oder Mischungen davon darstellt.

3. Mundpflegezusammensetzung nach Anspruch 1 oder Anspruch 2, wobei die Phosphatquelle wasserlöslich ist.

4. Mundpflegezusammensetzung nach irgendeinem der vorhergehenden Ansprüche, wobei das Calciumsilikat Calciumsilikathydrat ist, welches Hydrationswasser in einer Menge von 5 bis 50 Gewichts-% des Calciumsilikathydrats umfasst.

5. Mundpflegezusammensetzung nach irgendeinem der vorhergehenden Ansprüche, wobei das Calciumsilikat in einer Menge von 3 bis 50 Gewichts-% der Zusammensetzung, vorzugsweise von 5 bis 30 Gewichts-%, vorliegt.

6. Mundpflegezusammensetzung nach irgendeinem der vorhergehenden Ansprüche, wobei das Calciumsilikat und die lösliche Calciumquelle in einem Gewichtsverhältnis von 1:3 bis 10:1, vorzugsweise von 1:1,5 bis 5:1, vorliegen.

7. Mundpflegezusammensetzung nach irgendeinem der vorhergehenden Ansprüche, wobei die Phosphatquelle Trinatriumphosphat, Mononatriumdihydrogenphosphat, Dinatriumhydrogenphosphat, Ammoniumphosphat, Diammoniumhydrogenphosphat, Ammoniumdihydrogenphosphat, Trikaliumphosphat, Monokaliumdihydrogenphosphat, Dikaliumhydrogenphosphat oder eine Mischung davon, vorzugsweise Trinatriumphosphat, Mononatriumdihydrogenphosphat oder eine Mischung davon, darstellt.

8. Mundpflegezusammensetzung nach irgendeinem der vorhergehenden Ansprüche, wobei die Phosphatquelle in einer Menge von 0,5 bis 40 Gewichts-% der Zusammensetzung, vorzugsweise von 1 bis 30 Gewichts-%, vorliegt.

9. Mundpflegezusammensetzung nach irgendeinem der vorhergehenden Ansprüche, wobei die Zusammensetzung ferner ein Abscheidungshilfsmittel umfasst, das unter Calciumdihydrogenphosphat, Calciumsulfathemihydraten, Calciumaluminat, Calciummonohydrogenphosphat oder einer Mischung davon ausgewählt ist.

10. Mundpflegezusammensetzung nach Anspruch 9, wobei das Abscheidungshilfsmittel Calciumdihydrogenphosphat ist.

11. Mundpflegezusammensetzung nach Anspruch 9 oder Anspruch 10, wobei das Calciumsilikat und das Abscheidungshilfsmittel in einem Gewichtsverhältnis von 10:1 bis 1:1 vorliegen.

12. Mundpflegezusammensetzung nach irgendeinem der vorhergehenden Ansprüche, wobei die Mundpflegezusammensetzung eine einphasige wasserfreie Zusammensetzung ist.

13. Mundpflegezusammensetzung nach irgendeinem der vorhergehenden Ansprüche 1 bis 11, wobei die Zusammensetzung eine zweiphasige Zusammensetzung ist, umfassend eine Calciumphase und eine Phosphatphase, wobei das Calciumsilikat und die lösliche Calciumquelle in der Calciumphase vorliegen und die Phosphatquelle in der Phosphatphase vorliegen.

14. Mundpflegezusammensetzung nach Anspruch 13, wobei die Calciumphase und die Phosphatphase vor der Verwendung der Zusammensetzung physikalisch getrennt sind.

15. Zusammensetzung, wie in irgendeinem der Ansprüche 1 bis 14 beansprucht, zur Verwendung zum Reduzieren der Empfindlichkeit und/oder zum Remineralisieren der Zähne eines Individuums, umfassend den Schritt des Aufbringens der Zusammensetzung auf mindestens eine Oberfläche der Zähne des Individuums.

## Revendications

1. Composition de soin oral comprenant :
a) de 3 à 80 % en masse de silicate de calcium ;
b) une source de calcium soluble ;
c) une source de phosphate ; et
d) un support physiologiquement acceptable ;
dans laquelle le silicate de calcium et la source de calcium soluble sont présents dans un rapport de masse de 1:3 à 20:1.

2. Composition de soin oral selon la revendication 1, dans laquelle la source de calcium soluble est le chlorure de calcium, nitrate de calcium, acétate de calcium, lactate de calcium, gluconate de calcium, formate de calcium, malate de calcium, propionate de calcium, butyrate de calcium, bicarbonate de calcium, glycérophosphate de calcium, ascorbate de calcium ou mélanges de ceux-ci, de préférence chlorure de calcium, nitrate de calcium ou mélanges de ceux-ci.

3. Composition de soin oral selon la revendication 1 ou la revendication 2, dans laquelle la source de phosphate est soluble dans l'eau.

4. Composition de soin oral selon l'une quelconque des revendications précédentes, dans laquelle le silicate de calcium est un hydrate de silicate de calcium qui comprend de l'eau d'hydratation dans une quantité de 5 à 50 % en masse de l'hydrate de silicate de calcium.

5. Composition de soin oral selon l'une quelconque des revendications précédentes, dans laquelle le silicate de calcium est présent dans une quantité de 3 à 50 % en masse de la composition, de préférence de 5 à 30 %.

6. Composition de soin oral selon l'une quelconque des revendications précédentes, dans laquelle le silicate de calcium et la source de calcium soluble sont présents dans un rapport de masse de 1:3 à 10:1, de préférence de 1:1,5 à 5:1.

7. Composition de soin oral selon l'une quelconque des revendications précédentes, dans laquelle la source de phosphate est le phosphate de trisodium, dihydrogénophosphate de monosodium, hydrogénophosphate de disodium, phosphate d'ammonium, hydrogénophosphate de diammonium, dihydrogénophosphate d'ammonium, phosphate de tripotassium, dihydrogénophosphate de monopotassium, hydrogénophosphate de dipotassium ou un mélange de ceux-ci ; de préférence phosphate de trisodium, dihydrogénophosphate de monosodium ou un mélange de ceux-ci.

8. Composition de soin oral selon l'une quelconque des revendications précédentes, dans laquelle la source de phosphate est présente dans une quantité de 0,5 à 40 % en masse de la composition, de préférence de 1 à 30 %.

9. Composition de soin oral selon l'une quelconque des revendications précédentes, dans laquelle la composition comprend de plus un agent d'aide au dépôt choisi parmi le dihydrogénophosphate de calcium, des hémihydrates de sulfates de calcium, l'aluminate de calcium, le monohydrogénophosphate de calcium ou un mélange de ceux-ci.

10. Composition de soin oral selon la revendication 9, dans laquelle l'agent d'aide au dépôt est le dihydrogénophosphate de calcium.

11. Composition de soin oral selon la revendication 9 ou revendication 10, dans laquelle le silicate de calcium et l'agent d'aide au dépôt sont présents dans un rapport de masse de 10:1 à 1:1.

12. Composition de soin oral selon l'une quelconque des revendications précédentes, dans laquelle la composition de soin orale est une composition anhydre monophasique.

13. Composition de soin oral selon l'une quelconque des revendications 1 à 11 précédentes, dans laquelle la composition est une composition à phase double comprenant une phase de calcium et une phase de phosphate, dans laquelle le silicate de calcium et la source de calcium soluble sont présents dans la phase de calcium et la source de phosphate est présente dans la phase de phosphate.

14. Composition de soin oral selon la revendication 13, dans laquelle la phase de calcium et la phase de phosphate sont physiquement séparées avant l'utilisation de la composition.

15. Composition selon l'une quelconque des revendications 1 à 14 pour une utilisation pour réduire la sensibilité et/ou pour reminéraliser les dents d'un individu comprenant l'étape d'application de ladite composition sur au moins une surface des dents de l'individu.
